(19) Europäisches Patentamt

European Patent Office

Office européen des brevets

(11) **EP 1 242 042 B1**

(12) **EUROPÄISCHE PATENTSCHRIFT**

(45) Veröffentlichungstag und Bekanntmachung des Hinweises auf die Patenterteilung:
**10.05.2006 Patentblatt 2006/19**

(21) Anmeldenummer: **00993495.1**

(22) Anmeldetag: **13.12.2000**

(51) Int Cl.:
*A61K 8/24* (2006.01)    *A61K 8/25* (2006.01)
*A61K 8/26* (2006.01)    *A61Q 11/00* (2006.01)

(86) Internationale Anmeldenummer:
**PCT/EP2000/012656**

(87) Internationale Veröffentlichungsnummer:
**WO 2001/045659 (28.06.2001 Gazette 2001/26)**

(54) **ZAHNREINIGUNGSMITTEL**

MEANS FOR CLEANING TEETH

PRODUIT DE NETTOYAGE POUR LES DENTS

(84) Benannte Vertragsstaaten:
**AT BE CH CY DE DK ES FI FR GB GR IE IT LI LU MC NL PT SE TR**

(30) Priorität: **22.12.1999 DE 19961935**

(43) Veröffentlichungstag der Anmeldung:
**25.09.2002 Patentblatt 2002/39**

(73) Patentinhaber: **Henkel Kommanditgesellschaft auf Aktien**
**40589 Düsseldorf (DE)**

(72) Erfinder:
• **LEINEN, Hans, Theo**
  **40229 Düsseldorf (DE)**
• **DUSCHEK, Nicole**
  **40595 Düsseldorf (DE)**

(56) Entgegenhaltungen:
**DE-A- 3 425 152      GB-A- 1 414 678**
**GB-A- 2 106 783      US-A- 4 170 634**
**US-A- 4 863 722      US-A- 5 981 475**

**Beschreibung**

Die Erfindung betrifft ein Zahnreinigungsmittel in Form einer wäßrigen, pastösen oder flüssigen Dispersion mit einem Gehalt von 10-30 Gew.-% einer Poliermittelkombination und 20-50 Gew.-% an Feuchthaltemitteln, die als Poliermittel Kieselsäuren und Zirkoniumsilikat enthalten.

Zahnpasten werden bei der täglichen Reinigung der Zähne durch Bürsten mit einer Zahnbürste angewendet. Die Zahnpasta soll in erster Linie die Reinigung der Zahnoberflächen von Speiseresten, Verfärbungen durch z.B. Tabak oder Tee und von den fest anhaftenden bakteriellen Zahnbelägen, der sogenannten Plaque, unterstützen. Dies geschieht hauptsächlich durch die in der Zahnpasta enthaltenen Poliermittel, in geringerem Maße auch durch die enthaltenen Tenside. Die Poliermittel müssen, um ihre Reinigungs- und Polierwirkung zu entfalten, eine gewisse Abrasivität gegenüber der Zahnoberfläche aufweisen. Es ist jedoch von größter Bedeutung, daß die Abrasivität gegenüber Zahnschmelz und Dentin auf niedrigen Werten gehalten wird, um eine Schädigung der Zahnoberfläche durch den täglichen Gebrauch der Zahnpasta zu vermeiden. Vor allem dürfen die verwendeten Poliermittel keine tiefen Kratzer auf der Zahnoberfläche verursachen. Erwünscht ist vielmehr eine Einebnung der ggf. vorhandenen Rauhigkeit der Zahnoberfläche.

In US 4,863,722 sind Zahnpasten beschrieben, die wenigstens 22 Gew.-% Kieselsäurepoliermittel und 0,02 bis 0,08 Gew.-% Zirkoniumsilikat zur Verbesserung der Reinigungsleistung enthalten.

Aus DE 27 58 548 C2 waren Zahnpflegemittel mit einem Putzkörpergemisch aus einem Kieselsäurepoliermittel und einem calcinierten Aluminiumoxid als besonders wirksame Zahnreinigungsmittel bekannt. Zur Verminderung des Zahnschmelz-Abriebs wurde dort ein Zusatz von bestimmten anorganischen Elektrolytsalzen empfohlen. Trotzdem wurden nur äußerst hohe Dentinabriebwerte (RDA) und Schmelzabriebwerte (REA) im Bereich von 300 - 500 erreicht.

Aus DE 34 25 152 war eine Poliermittelkombination aus einem Kieselsäure-Poliermittel und einem schwach calcinierten Aluminiumoxid bekannt, mit welcher sich die Abrasivität bei guter Reinigungsleistung deutlich senken ließ.

Es wurde nun gefunden, daß sich die Reinigungsleistung solcher Zahnpasten noch weiter steigern läßt, ohne daß die Abrasivität und Kratzwirkung erhöht werden, wenn man diesen Zahnpasten eine relativ kleine Menge an Zirkoniumsilikat zusetzt.

Gegenstand der Erfindung sind daher Zahnreinigungsmittel in Form einer wäßrigen, pastösen oder flüssigen Dispersion mit einem Gehalt von

10-30 Gew.-% einer Poliermittelkombination und

20-50 Gew.-% Feuchthaltemitteln

dadurch gekennzeichnet, daß als Poliermittel Kieselsäuren (a), Aluminiumoxid (b) und Zirkoniumsilikat (c) im Gewichtsverhältnis (a) : (b) : (c) = 10 : (0,1-2) : (0,01-0,5) enthalten sind.

Dabei hat sich gezeigt, daß bei einer Erhöhung der Konzentration des Zirkoniumsilikats gegenüber der Kieselsäure nicht nur die Abrasivität und Kratzwirkung zunimmt, sondern die Reinigungswirkung wieder abnimmt.

Geeignete Kieselsäure-Poliermittel sind z.B. Gelkieselsäuren, die durch Umsetzung von Natriumsilikatlösungen mit starken wäßrigen Mineralsäuren unter Ausbildung eines Hydrosols, Alterung zum Hydrogel, Waschen und Trocknen erzeugt werden. Erfolgt die Trocknung unter schonenden Bedingungen auf Wassergehalte von 15 - 35 Gew.-%, so werden sogenannte Hydrogelkieselsäuren erhalten, wie sie z.B. aus US 4,153,680 bekannt sind.

Durch Trocknung auf Wassergehalte von unterhalb 15 Gew.-% erfolgt eine irreversible Schrumpfung der vorher lockeren Struktur des Hydrogels zur dichten Struktur des sogenannten Xerogels. Solche Xerogelkieselsäuren sind z.B. in US 3,538,230 beschrieben.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Herstellung von Fällungskieselsäuren sind z.B. in DE-OS 25 22 486 und in DE-OS 31 14 493 beschrieben. Bevorzugt geeignet sind z.B. Fällungskieselsäuren, die eine mittlere Teilchengröße von 5 - 20 $\mu$m, einen Siebrückstand 45$\mu$m von weniger als 1 Gew.-% und eine spezifische Oberfläche (BET) von 100 - 300 m$^2$/g aufweisen.

Als Aluminiumoxid-Poliermittel eignet sich bevorzugt eine schwach calcinierte Tonerde mit einem Gehalt von wenigstens 10 Gew.-%, von $\alpha$-Aluminiumoxid verschiedener, sogenannter $\gamma$-Aluminiumoxid-Modifikationen.

Geeignete schwach calcinierte Tonerden werden durch Calcination aus Aluminiumhydroxid hergestellt. Aluminiumhydroxid geht durch Calcination in das bei Temperaturen oberhalb 1200°C thermodynamisch stabile $\alpha$-Al$_2$O$_3$ über. Die bei Temperaturen zwischen 400 und 1000°C auftretenden, thermodynamisch instabilen Al$_2$O$_3$-Modifikationen bezeichnet man als Gamma-Formen (vgl. Ullmann, Enzyklopädie der technischen Chemie, 4. Auflage (1974), Band 7, Seite 298). Durch Wahl der Temperatur und der Zeitdauer bei der Calcination kann man den Calcinationsgrad, d.h. die Umwandlung in das thermodynamisch stabile $\alpha$-Al$_2$O$_3$ auf beliebige Höhe einstellen. Man erhält durch schwache Calcination eine Tonerde mit einem Gehalt an $\gamma$-Al$_2$O$_3$, der um so niedriger ist, je höher die Calcinationstemperatur und je länger die Calcinationsdauer gewählt wird. Schwach calcinierte Tonerden unterscheiden sich von reinem $\alpha$-Al$_2$O$_3$ durch eine geringere Härte der Agglomerate, eine größere spezifische Oberfläche und größere Porenvolumina.

Der Dentinabrieb (RDA) der erfindungsgemäß zu verwendenden schwächer calcinierten Tonerden mit einem Anteil von 10 - 50 Gew.-% $\gamma$-Al$_2$O$_3$ beträgt nur 30 - 60 % des Dentinabriebs eines stark calcinierten, reinen $\alpha$-Al$_2$O$_3$ (gemessen in einer Standard-Zahnpaste mit 20 Gew.-% Tonerde als einzigem Poliermittel).

Im Gegensatz zu $\alpha$-Al$_2$O$_3$ läßt sich das $\gamma$-Al$_2$O$_3$ mit einer wäßrig-ammoniakalischen Lösung von Alizarin S (1,2-Dihydroxy-9,10-anthrachinon-4-sulfonsäure) rot anfärben. Man kann den Grad der Anfärbbarkeit als Maß für den Calcinationsgrad bzw. für den Anteil an $\gamma$-Al$_2$O$_3$ in einer calcinierten Tonerde wählen :

Ca. 1 g Al$_2$O$_3$, 10 ml einer Lösung von 2 g/l Alzarin S in Wasser und 3 Tropfen einer wäßrigen, 10 Gew.-%igen Lösung von NH$_3$ werden in ein Reagenzglas gegeben und kurz aufgekocht. Das Al$_2$O$_3$ wird anschließend abfiltriert, nachgewaschen, getrocknet und unter dem Mikroskop beurteilt oder farbmetrisch ausgewertet.

Geeignete, schwach calcinierte Tonerden mit einem Gehalt von 10 - 50 Gew.-% $\gamma$-Al$_2$O$_3$ lassen sich nach diesem Verfahren schwach bis tief rosa anfärben.

Aluminiumoxid-Poliermittel verschiedener Calcinationsgrade, Mahlfeinheit und Schüttgewichte sind im Handel erhältlich, z.B. die "Poliertonerden" der Firma Giulini-Chemie beziehungsweise ALCOA.

Eine bevorzugt geeignete Qualität "Poliertonerde P10 feinst" weist eine Agglomeratgröße unter 20 $\mu$m, eine mittlere Primärkristallgröße von 0,5 - 1,5 $\mu$m und ein Schüttgewicht von 500 - 600 g/l auf.

Zirkoniumsilikat kommt in relativ hoher Reinheit als Zirkon (ZrSiO$_4$) in den Zirkonsanden an den Küsten von Australien und Florida vor. Im Handel sind Qualitäten mit Teilchengrößen unter 44 $\mu$m und mit Verunreinigungen von weniger als 5 Gew.-% an anderen Silikaten. Bevorzugte Teilchengrößen sind die von 0,5-1,5 $\mu$m, die in den verwendeten Qualitäten mehr als 50 Gew.-% ausmachen sollten.

Eine weitere Steigerung der Reinigungswirkung der erfindungsgemäßen Zahnreinigungsmittel kann durch einen Zusatz an kondensierten Phosphaten erreicht werden. In einer bevorzugten Ausführung enthalten die erfindungsgemäßen Zahnreinigungsmittel zur Erhöhung der Reinigungswirkung ein kondensiertes Phosphat, bevorzugt ein solches aus der Gruppe Tripolyphosphat, Pyrophosphat oder Trimetaphosphat in Form eines Alkali- oder Ammoniumsalzes in einer Menge von 1-12 Gew.-%.

Die kondensierten Phosphate sind in Form ihrer Alkalisalze, bevorzugt in Form ihrer Natrium- oder Kaliumsalze enthalten. Die wäßrigen Lösungen dieser Phosphate reagieren aufgrund hydrolytischer Effekte alkalisch. Durch Säurezusatz wird der pH-Wert der erfindungsgemäßen Zahnpflegemittel auf Werte von 7,5 - 9 eingestellt. Als Säuren können dabei z.B. Zitronensäure, Phosphorsäure oder saure Salze, z.B. NaH$_2$PO$_4$ verwendet werden. Man kann aber auch anteilig saure Salze der kondensierten Phosphate, also z.B. K$_2$H$_2$P$_2$O$_7$ mitverwenden, um den gewünschten pH-Wert des Zahnpflegemittels einzustellen.

Es können auch Gemische verschiedener kondensierter Phosphate oder auch hydratisierte Salze der kondensierten Phosphate eingesetzt werden. Die spezifizierten Mengen von 2 - 12 Gew.-% beziehen sich jedoch auf die wasserfreien Salze. Bevorzugt ist als kondensiertes Phosphat ein Natrium- oder Kalium-Tripolyphosphat in einer Menge von 5 - 10 Gew.-% der Zusammensetzung enthalten.

Als Feuchthaltemittel können wasserlösliche Polyole wie z.B. Sorbit, Xylit, Glycerin, Propylenglycol, Polyethylenglycole mit Molekulargewichten von 400-2000 oder Gemische von zwei oder mehreren dieser Verbindungen enthalten sein. Bevorzugt sind diese Feuchthaltemittel in einer Konzentration von 25-40 Gew.-% enthalten.

Eine weitere Verbesserung der Reinigungswirkung der erfindungsgemäßen Zahnreinigungsmittel kann man durch Zusatz eines geeigneten Tensids erzielen. Der Tensidzusatz kann auch zur Erzeugung eines Schaums beim Zähnebürsten, zur Stabilisierung der Poliermitteldispersion sowie zur Emulgierung oder Solubilisierung der Aromaöle erwünscht sein. Geeignete Tenside, die eine gewisse Schaumwirkung entfalten, sind die anionischen Tenside, z.B. Natriumalkylsulfate mit 12 - 18 C-Atomen in der Alkylgruppe. Diese Tenside weisen auch eine gewisse enzymhemmende Wirkung auf, den bakteriellen Stoffwechsel des Zahnbelags. Weitere geeignete Tenside sind Alkalisalze, bevorzugt Natriumsalze von Alkylpolyglycolethersulfat mit 12 - 16 C-Atomen in der linearen Alkylgruppe und 2 - 6 Glycolethergruppen im Molekül, von linearem Alkan-(C$_{12}$-C$_{18}$)-sulfonat, von Sulfobernsteinsäuremonoalkyl-(C$_{12}$-C$_{18}$)-estern, von sulfatierten Fettsäuremonoglyceriden, sulfatierten Fettsäurealkanolamiden, Sulfoessigsäurealkyl-(C$_{12}$-C$_{16}$)-estern, Acylsarcosinen, Acyltauriden und Acylisethionaten mit jeweils 8 - 18 C-Atomen in der Acylgruppe.

Auch zwitterionische und ampholytische Tenside können, bevorzugt in Kombination mit anionischen Tensiden, eingesetzt werden.

Besonders bevorzugt zur Förderung der Reinigungswirkung ist aber der Einsatz nichtionogener Tenside. Geeignete nichtionische Tenside sind z.B. die Anlagerungsprodukte von Ethylenoxid an Fettalkohole, an Fettsäuren, an Fettsäuremonoglyceride, an Sorbitan-Fettsäuremonoester oder an Methylglucosid-Fettsäuremonoester. Die angelagerte Menge an Ethylenoxid sollte dabei so hoch sein, daß die Tenside wasserlöslich sind, d.h. es sollte wenigstens 1 g/l in Wasser bei 20°C löslich sein.

Eine weitere Gruppe von geeigneten Tensiden sind die Alkyl-(oligo)-glycoside mit 8 - 16 C-Atomen in der Alkylgruppe und einem Oligomerisationsgrad des Glycosidrestes von 1 - 4. Alkyl-(oligo)-glycoside, ihre Herstellung und Verwendung als oberflächenaktive Stoffe sind z.B. aus US-A-3,839,318, DE-A-20 36 472, EP-A-77 167 oder WO-A-93/10132 bekannt. Bezüglich des Glycosidrestes gilt, daß sowohl Monoglycoside (x = 1), bei denen ein Monosaccharidrest glycosidisch an einen Fettalkohol mit 10 bis 16 C-Atomen gebunden ist, als auch oligomere Glycoside mit einem Oligomerisationsgrad x bis 10 geeignet sind. Der Oligomerisationsgrad ist dabei ein statistischer Mittelwert, dem eine für solche technischen Produkte übliche Homologenverteilung zugrunde liegt.

Bevorzugt eignet sich als Alkyl-(oligo)-glycosid ein Alkyl-(oligo)-glucosid der Formel $RO(C_6H_{10}O)_x$-H, in der R eine Alkylgruppe mit 12 bis 14 C-Atomen ist und x einen Mittelwert von 1 bis 4 hat.

Insbesondere zur Solubilisierung der meist wasserunlöslichen Aromaöle kann ein nichtionogener Lösungsvermittler aus der Gruppe der oberflächenaktiven Verbindungen erforderlich sein. Besonders geeignet für diesen Zweck sind z.B. oxethylierte Fettsäureglyceride, oxethylierte Fettsäure-sorbitanpartialester oder Fettsäurepartialester von Glycerin- oder Sorbitan-Oxethylaten. Lösungsvermittler aus der Gruppe der oxethylierten Fettsäureglyceride umfassen vor allem Anlagerungsprodukte von 20 bis 60 Mol Ethylenoxid an Mono- und Diglyceride von linearen Fettsäuren mit 12 bis 18 C-Atomen oder an Triglyceride von Hydroxyfettsäuren wie Oxystearinsäure oder Ricinolsäure. Weitere geeignete Lösungsvermittler sind oxethylierte Fettsäuresorbitanpartialester; das sind bevorzugt Anlagerungsprodukte von 20 bis 60 Mol Ethylenoxid an Sorbitanmonoester und Sorbitandiester von Fettsäuren mit 12 bis 18 C-Atomen. Ebenfalls geeignete Lösungsvermittler sind Fettsäurepartialester von Glycerin- oder Sorbitan-Oxethylaten; das sind bevorzugt Mono- und Diester von $C_{12}$-$C_{18}$-Fettsäuren und Anlagerungsprodukten von 20 bis 60 Mol Ethylenoxid an 1 Mol Glycerin oder an 1 Mol Sorbit.

Die erfindungsgemäßen Zahnreinigungsmittel enthalten bevorzugt als Lösungsvermittler für gegebenenfalls enthaltene Aromaöle Anlagerungsprodukte von 20 bis 60 Mol Ethylenoxid an gehärtetes oder ungehärtetes Ricinusöl (d.h. an Oxystearinsäure- oder Ricinolsäure-triglycerid), an Glycerin-mono- und/oder -distearat oder an Sorbitanmono- und/oder -distearat.

Als Geschmacksstoffe sind z.B. Süßungsmittel und/oder Aromaöle enthalten. Als Aromaöle kommen alle für Mund- und Zahnpflegemittel gebräuchlichen natürlichen und synthetischen Aromen in Frage. Natürliche Aromen können sowohl in Form der aus den Drogen isolierten etherischen Öle als auch der aus diesen isolierten Einzelkomponenten verwendet werden. Bevorzugt sollte wenigstens ein Aromaöl aus der Gruppe Pfefferminzöl, Krausenminzöl, Anisöl, Sternanisöl, Kümmelöl, Eukalyptusöl, Fenchelöl. Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte synthetisch erzeugten Komponenten dieser Öle enthalten sein. Die wichtigsten Komponenten der genannten Öle sind z.B. Menthol, Carvon, Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z.B. Menthylacetat, Vanillin, Jonone, Linalylacetat, Rhodinol und Piperiton.

Darüber hinaus können die erfindungsgemäßen Zahnreinigungsmittel einen therapeutischen Wirkstoff zur Bekämpfung von Karies, Zahnstein, Parodontitis oder anderer Mund- oder Zahnerkrankungen enthalten. Ein bevorzugt enthaltener Wirkstoff ist eine karieshemmende Fluorverbindung, bevorzugt aus der Gruppe der Fluoride oder Monofluorophosphate in einer Menge von 0,1 - 0,5 Gew.-% Fluor. Geeignete Fluorverbindungen sind z.B. Natriumfluorid, Kaliumfluorid, Zinnfluorid, Natriummonofluorophosphat ($Na_2PO_3F$), Kaliummonofluorophosphat oder das Fluorid einer organischen Aminoverbindung.

Bevorzugt geeignete therapeutische Wirkstoffe sind z.B. Antizahnstein-Wirkstoffe, z.B. Organophosphonate wie 1-Azacycloheptan-2,2-diphosphonsäure (Na-Salz) oder 1-Hydroxyethan-1,1-diphosphonsäure (Na-Salz) oder antimikrobielle Plaque-Inhibitoren wie z.B. Hexachlorophen, Triclosan, Bromchlorophen, Phenyl-salicylsäureester. Auch Stoffe, die eine remineralisierungsfördernde und den Verschluß von Dentalläsionen fördernde Wirkung haben, wie z.B. Dicalciumphosphat-dihydrat, bevorzugt in Kombination mit Magnesiumionen, können in den erfindungsgemäßen Zahnpasten enthalten sein.

Schließlich können die erfindungsgemäßen Zahnpasten noch weitere Komponenten enthalten, die in Zahnpflegemitteln üblich sind und die erfindungsgemäßen Effekte nicht verringern. Solche üblichen Zusätze in Zahnpasten sind z.B.:

- weitere Poliermittel in kleineren Mengen von z.B. 1 - 5 Gew.-%, z.B. Calciumcarbonat (Kreide) Dicalciumphosphat-dihydrat, unlösliches Natriummetaphosphat, Calciumpyrophosphat, Hydroxylapatit, Aluminiumhydroxid, Natriumaluminiumsilikate (Zeolith A) oder teilchenförmige organische Polymere, z.B. Polymethacrylat,
- Pigmente, z.B. Titandioxid oder Zinkoxid,
- Farbstoffe
- pH-Stellmittel und Puffersubstanzen, z.B. Natriumcitrat oder Natriumbicarbonat, Natriumbenzoat,
- wundheilende und entzündungshemmende Stoffe wie z.B. Allantoin, Harnstoff, Panthenol, Azulen oder Kamillenextrakt,
- Konservierungsstoffe wie z.B. Sorbinsäure-Salze, p-Hydroxybenzoesäure-Ester.

Die folgenden Beispiele sollen den Erfindungsgegenstand näher erläutern:

Beispiele:

Es wurden folgende Zahnpasten hergestellt:

| | V | 1 | 2 |
|---|---|---|---|
| Zeodent® 113 | 14,0 | 14,0 | 14,0 |
| Zirkoniumsilikat | - | 0,05 | 0,10 |
| Poliertonerde P10 feinst | 1,0 | 1,0 | 1,0 |
| $Na_5P_3O_{10}$(Na-Tripolyphosphat) | 5,0 | 5,0 | 5,0 |
| Na F | 0,24 | 0,24 | 0,24 |
| Na-Saccharinat | 0,20 | 0,20 | 0,20 |
| PHB-Methylester | 0,10 | 0,10 | 0,10 |
| Titandioxid | 0,50 | 0,50 | 0,50 |
| Cekol® 700H (CMC) | 1,25 | 1,25 | 1,25 |
| Sorbit (70%-ig) | 32,0 | 32,0 | 32,0 |
| 1,2-Propylenglycol | 3,0 | 5,0 | 3,0 |
| Polyethylenglycol 400 | 2,0 | - | 2,0 |
| Tagat R60 | 1,0 | 1,0 | 1,0 |
| Aromaöl | 0,85 | 0,85 | 0,85 |
| Wasser | 38,86 | 38,81 | 38,76 |
| **CRS (%)** | **78** | **91** | **86** |

Es wurden folgende Handelsprodukte eingesetzt:

| | |
|---|---|
| Zeodent® 113: (Huber Chemicals) | Kieselsäure-Poliermittel Mittlere Teilchengröße: 12 $\mu$m |
| Zirconiumsilicate: (TAM-Ceramics Inc.) | Zirkonsilikat: min. 95 Gew.-% |
| | Staurolith: max. 5 Gew.-% |
| | Partikelgröße: 98 Gew.-% L 44 $\mu$m |
| Cekol® 700 H: (Metsae Serla) | Carboxymethylcellulose Viskosität (2 %-ig): 600-1000 m·Pa·s (20°C) |
| Tagat® R60: (Goldschmidt) | Hydr. Rizinusöl + 60 Mol EO |

**Bestimmung der Reinigungswirkung (CRS)**

**Prüfmethode:**

Die Oberfläche von Rinderzähnen wurde konditioniert, unter definierten Bedingungen mit Tee eingefärbt und unter definierten Bedingungen mit der zu prüfenden Zahnpaste gereinigt. Die dabei erzielte Aufhellung wurde farbmetrisch untersucht und mit der Aufhellung verglichen, die mit einer Standard-Prüfpaste erhalten wurde.

**Probenvorbereitung**

Rinder-Schneidezähne wurden in Blöcke von 7 x 7 mm geschnitten und die Blöcke mit Wachs so auf Plexiglasquader (1 x 2,5 x 2,5 cm) montiert, daß nur die Schmelzoberfläche frei blieb. Die Schmelzoberfläche wurde poliert bis sie einheitlich glatt erschien.

**Probenkonditionierung**

Die montierten Zahnblöcke wurden nacheinander in 0,12 N Salzsäure (60 sec.), gesättigte $Na_2CO_3$-Lösung (120 sec.) und in 1 %ige Phytinsäure-Lösung (60 sec.) eingetaucht. Nach jeder Behandlung wurden die Proben mit entsalztem Wasser gespült und mit saugfähigem Papier trocken getupft. Die Zahnproben wurden dann in das Anschmutzungsgerät eingehängt und 5 Tage lang durch eine Lösung von schwarzem Tee von 20°C bewegt. Die Teelösung wurde durch Extraktion eines 1,5 g Teebeutels mit 300 g kochenden Wassers (10 Min.) zubereitet und zweimal täglich erneuert.

**Putzversuche**

Die montierten Zahnblöcke wurden dann in eine Grabenstetter V-8-Bürstmaschine eingebracht und in der Zahnpasten-Aufschlämmung aus 20 g Paste und 40 g entsalztem Wasser mit Hilfe einer weichen Oral-B-Zahnbürste mit 150 g Anpreßdruck gebürstet.Als Putz-Standard wurde eine Aufschlämmung von 10 g Calciumpyrophosphat in 50 g einer Carboxymethylcellulose-Quellung (0,5 % CMC, 10 % Glycerin, 89,5 % Wasser) verwendet. Die Reinigungswirkung dieses Standards wird als 100 %-Wert definiert.

**Messung der Reinigungswirkung**

Die Messungen der Aufhellung wurden mit Hilfe eines Dr. Lange Farbdifferenz-Meßgeräts (Typ Micro Color (DC 8334)) gemäß der Deutschen Industrienorm DIN 5033 durchgeführt. Es wurde eine Xenon-Lampe verwendet, die Normallicht D 65, entsprechend dem Tageslicht erzeugt. Als Farbstandard diente Bariumsulfat.
Es wurden zweifache Messungen eines kreisförmigen Bereiches von 7 mm Durchmesser auf der Probenoberfläche durchgeführt. Für jede Zahnpatenprobe wurden 8 angefärbte Zahnproben verwendet und Mittelwerte gebildet. Als Prüfparameter wurde das nach DIN 5033 festgelegte Inkrement Y verwendet, das als Maß für die Helligkeit einer Farbe gilt.
Die Helligkeitsmessung erfolgte nach jeweils 1000 Bürsthüben. Die Reinigungswirkung CRS (cleaning ratio soft) ergibt sich aus

$$CRS\,[\,\%\,] = \frac{\text{mittleres Inkrement Y Test-Paste}}{\text{mittleres Inkrement Y Putzstandard}} \cdot 100\,\,[\,\%\,]$$

Die Ergebnisse sind in der Tabelle aufgeführt.

**Patentansprüche**

1. Zahnreinigungsmittel in Form einer wäßrigen, pastösen oder flüssigen Dispersion mit einem Gehalt von
   10-30 Gew.-% einer Poliermittelkombination und
   20-50 Gew.-% Feuchthaltemitteln
   **dadurch gekennzeichnet, daß** als Poliermittel Kieselsäuren (a), Aluminiumoxid (b) und Zirkoniumsilikat (c) Gewichtsverhältnis (a) : (b) : (c) = 10:(0,1 - 2) : (0,01 - 0,5) enthalten sind.

2. Zahnreinigungsmittel und Anspruch 1, **dadurch gekennzeichnet, daß** zur Erhöhung der Reinigungswirkung ein kondensiertes Phosphat aus der Gruppe Tripolyphosphat, Pyrophosphat oder Trimetaphosphat in Form eines Alkali- oder Ammoniumsalzes in einer Menge von 1-12 Gew.-% enthalten ist.

3. Zahnreinigungsmittel und Anspruch 1 oder 2, **dadurch gekennzeichnet, daß** als Feuchthaltemittel Sorbit, Glycerin, Propylenglycol-1,2, Polyethylenglycol oder ein Gemisch aus zwei oder mehreren dieser Verbindungen enthalten ist.

4. Zahnreinigungsmittel nach Anspruch 1-3, **dadurch gekennzeichnet, daß** als Aluminiumoxid eine schwach calcinierte Tonerde mit einem Gehalt von wenigstens 10 Gew.-% von Alpha-Aluminiumoxid verschiedener $Al_2O_3$-Modifikationen enthalten ist.

5. Zahnreinigungsmittel nach einem der Ansprüche 1 - 4, **dadurch gekennzeichnet, daß** als Feuchthaltemittel Sorbit in einer Menge von 25 - 40 Gew.-% enthalten ist.

6. Zahnreinigungsmittel nach einem der Ansprüche 1 - 5, **dadurch gekennzeichnet, daß** als kondensiertes Phosphat 5 - 10 Gew.-% Natrium- oder Kalium-Tripolyphosphat enthalten ist.

7. Zahnreinigungsmittel nach einem der Ansprüche 1 - 6, **dadurch gekennzeichnet, daß** als Kieselsäurepoliermittel eine Fällungskieselsäure mit einer Teilchengröße von 5 - 20 μm und einer spezifischen Oberfläche (BET) von 100 - 300 g/l enthalten ist.

8. Zahnreinigungsmittel nach einem der Ansprüche 1 - 7, **dadurch gekennzeichnet, daß** eine Fluorverbindung, bevorzugt aus der Gruppe der Fluoride oder Monofluorophosphate in einer Menge von 0,1 - 0,5 Gew.-% Fluor enthalten ist.

9. Zahnreinigungsmittel nach einem der Ansprüche 1-8, **dadurch gekennzeichnet, daß** Antizahnsteinwirkstoffe aus der Gruppe der Organophosphate und/oder antimikrobielle Plaque Inhibitoren, insbesondere Triclosan, enthalten sind.

**Claims**

1. A means for cleaning teeth in the form of an aqueous, pasty or liquid dispersion with a content of
   10-30% by weight of a polishing agent combination and
   20-50% by weight of moisture-retention agents
   **characterized in that** the polishing agents present are silicas (a), aluminum oxide (b) and zirconium silicate (c) [lacuna] weight ratio (a):(b):(c) = 10: (0.1-2) : (0.01-0.5).

2. The means for cleaning teeth and claim 1, **characterized in that**, to increase the cleaning action, a condensed phosphate from the group tripolyphosphate, pyrophosphate or trimetaphosphate in the form of an alkali metal salt or ammonium salt in an amount of 1-12% by weight is present.

3. The means for cleaning teeth and claim 1 or 2, **characterized in that** the moisture-retention agent present is sorbitol, glycerol, 1,2-propylene glycol, polyethylene glycol or a mixture of two or more of these compounds.

4. The means for cleaning teeth as claimed in claim 1-3, **characterized in that** the aluminum oxide present is a weakly calcined clay earth with a content of at least 10% by weight of α-aluminum oxide of various $Al_2O_3$ modifications.

5. The means for cleaning teeth as claimed in any of claims 1-4, **characterized in that** the moisture-retention agent is present is sorbitol in an amount of 25-40% by weight.

6. The means for cleaning teeth as claimed in any of claims 1-5, **characterized in that** the condensed phosphate present is 5-10% by weight of sodium or potassium tripolyphosphate.

7. The means for cleaning teeth as claimed in any of claims 1-6, **characterized in that** the silica polishing agent present is a precipitated silica with a particle size of 5-20 μm and a specific surface area (BET) of 100-300 g/l.

8. The means for cleaning teeth as claimed in any of claims 1-7, **characterized in that** a fluorine compound, preferably from the group of fluorides or monofluorophosphates, is present in an amount of 0.1-0.5% by weight of fluorine.

9. The means for cleaning teeth as claimed in any of claims 1-8, **characterized in that** antitartar active ingredients from the group of organophosphates and/or antimicrobial plaque inhibitors, in particular triclosan, are present.

**Revendications**

1. Produit de nettoyage pour les dents sous forme de dispersion aqueuse, pâteuse ou liquide ayant une teneur de
   10-30 % en poids d'une combinaison d'agents de polissage, et
   20-50 % en poids d'un agent de maintien de l'humidité,
   **caractérisé en ce que** comme agent de polissage, il contient des acides siliciques (a), de l'oxyde d'aluminium (b) et du silicate de zirconium (c) dans un rapport en poids de (a) : (b) : (c) = 10 : (0,1 - 2) : (0,01 - 0,5).

2. Produit de nettoyage pour les dents selon la revendication 1, **caractérisé en ce que**, pour renforcer l'action de nettoyage, il contient un phosphate condensé appartenant au groupe des tripolyphosphates, pyrophosphates ou trimétaphosphates sous la forme d'un sel d'alcali ou d'ammonium dans une proportion de 1 - 12 % en poids.

**3.** Produit de nettoyage pour les dents selon la revendication 1 ou 2, **caractérisé en ce que** comme agent de maintien de l'humidité, il contient du sorbitol, de la glycérine, du propylèneglycol-1,2, du polyéthylèneglycol ou un mélange de deux ou plusieurs de ces composés.

**4.** Produit de nettoyage pour les dents selon l'une quelconque des revendications 1 à 3, **caractérisé en ce que** comme oxyde d'aluminium, il contient une alumine légèrement calcinée ayant une teneur d'au moins 10 % en poids d'oxyde d'aluminium alpha de diverses variantes d'$Al_2O_3$.

**5.** Produit de nettoyage pour les dents selon l'une quelconque des revendications 1 à 4, **caractérisé en ce que** comme agent de maintien de l'humidité, il contient du sorbitol dans une proportion de 25-40 % en poids.

**6.** Produit de nettoyage pour les dents selon l'une quelconque des revendications 1 - 5, **caractérisé en ce que** comme phosphate condensé, il contient 5 - 10 % en poids de tripolyphosphate de sodium ou de potassium.

**7.** Produit de nettoyage pour les dents selon l'une quelconque des revendications 1 - 6, **caractérisé en ce que** comme agent de polissage à base d'acides siliciques, il contient un acide silicique précipité ayant une taille des particules de 5-20 $\mu$m et une surface spécifique (BET) de 100-300 g/l.

**8.** Produit de nettoyage pour les dents selon l'une quelconque des revendications 1 - 7, **caractérisé en ce qu'**il contient un composé fluoré, de préférence appartenant au groupe des fluorures ou des monofluorophosphates dans une proportion de 0,1 -0,5 % en poids, calculé en tant que fluor.

**9.** Produit de nettoyage pour les dents selon l'une quelconque des revendications 1 - 8, **caractérisé en ce qu'**il contient des principes actifs anti-tartre appartenant au groupe des organophosphates et/ou des inhibiteurs de plaque anti-microbiens, en particulier du triclosan.